# EUROPEAN PATENT APPLICATION

(11) **EP 2 609 957 A1**
(43) Date of publication of application: **03.07.2013**
(21) Application number: 12176472.4
(22) Date of filing: 13.07.2012
(51) Int. Cl.: A61M 25/00, A61M 27/00, A61M 1/00

(54) **Drainage system for body fluids**

(30) Priority: 29.12.2011 IT TO20111231
(71) Applicant: Surgika S.r.l., 52027 San Giovanni, Valdarno (IT)
(72) Inventor: Feri, Marco, 52100 Arezzo (IT); Marini, Andrea, 52028 Terranuova Bracciolini (IT)
(74) Representative: Bergadano, Mirko

(57) **Abstract**

There is disclosed a drainage system (1) for body fluids comprising a catheter (2) having a distal segment (2a) adapted to be inserted within the body of a patient and a proximal segment (2b) arranged in use outside the body of the patient, a plurality of openings (4) for the passage of the fluids from the outside of the catheter (2) to the inside of the catheter (2), a spacing device (3) of the catheter (2) from the tissues of the patient, which comprises an expandable balloon (8) arranged helixwise about the distal segment (2a) laterally with respect to the openings (4). The catheter (2) defines, on the relative external surface, a groove (5) adapted to at least partially accommodate the expandable balloon (8). There is also disclosed a fastening device (10) for a drainage system (1) comprising a fastening element (11) forming a through-seat (14) for inserting the catheter (2) and connectable to the skin (7) of the patient, and a retaining element (12) releasably coupleable to the fastening element (11) to lock the position of the catheter (2) on the fastening element (11).

## Description

The present invention relates to a drainage system for body fluids and to a fastening device for such a drainage system.

In the following description, reference will be specifically made to a thoracic drainage system, although this is in no way intended to limit the scope of protection defined by the appended claims. In particular, there is intended that the drainage system of the invention can also be conveniently used for other types of drainage, for example abdominal drainage, gastric drainage and drainage of infected sites. The drainage system can also be used in veterinary medicine.

In the human body, the lungs are surrounded by the pleura, a serous two-layered membrane, which coats and adheres to the internal wall of the thorax with its parietal layer and to the distal wall of each lung with its visceral layer. The two layers are continuous to one another and adhere closely thus defining a space, designated pleural cavity, which contains a lubricating fluid that allows the two layers to slide on one another during breathing.

Following injury, surgery or as a consequence of a disease, a condition can arise that leads to the accumulation of excess liquid, gas or blood in the pleural cavity. This accumulation leads to the patient not being able to breathe correctly due to a decreased ability to expand the lungs.

In order to remove excess fluid, a drainage system must be used.

The drainage systems currently on the market are formed by a catheter made of flexible polymeric material, which is partially positioned inside the thoracic cavity and partially extends outside the patient. For clarity, the portion of catheter which in use is outside the thoracic cavity will be designated distal segment, whereas the portion of catheter which in use extends outside the patient will be designated proximal segment.

To promote the drainage of the fluid, the catheter may have not only one opening at the distal end, but also a series of side openings along the distal end segment of the catheter.

US2004/0087905 discloses a thoracic drainage device that has a plurality of drainage openings in the distal segment of the catheter. This kind of device has, however, the drawback that the openings are easily obstructed by the soft tissues which adhere to the catheter and prevent the effective drainage of the fluid.

The thoracic drainage system is generally positioned surgically by cutting a small incision in the skin of the patient and inserting the drainage system within the thoracic cavity by using a pointed needle which passes inside the catheter and can be extracted after the drainage system has been positioned.

Once inserted within the thoracic cavity of the patient, the drainage system must be fastened to the body of the patient so as not to come out from the thorax and so as not to move causing damages to internal organs as well as pain to the patient.

In the solution shown in US2004/0087905, the fastening of the drainage catheter to the thoracic wall of the patient is achieved by the expansion of a balloon inserted within the thoracic cavity and by means of a extracorporeal clip locking device.

This fastening solution has several drawbacks.

First, the balloon arranged within the thoracic cavity provides a limited fixing area with respect to the length of the drainage catheter; the segment of catheter which extends within the thorax is therefore free to move and can be displaced thus risking to cause damages to the internal organs.

Further, to effectively lock the catheter, the balloon must be rather large; the presence of a bulky balloon inside the body causes a considerable discomfort to the patient.

A further disadvantage of this kind of solution is that, even though the clip device has a larger diameter than the diameter of the catheter and the length of the incision in the skin, and even though it has rounded edges at the areas in contact with the body of the patient, the device is not firmly fastened to the body of the patient and can therefore move thus causing discomfort and pain and risking to widen the incision or catch the skin of the patient.

Finally, the combination of the balloon and clip device allows a certain clearance for the catheter and results in the latter being fastened only at the incision in the skin of the patient. This concentrates the whole fastening load in the area which is already sensitive and fragile and results in an easy tearing of the skin with a subsequent displacement of the drainage catheter.

US2008/0021382 discloses a drainage system according to the preamble of claim 1.

It is an object of the present invention to therefore provide a drainage system which solves at least one of the above cited drawbacks in a simple and cost-effective manner.

The above said object is solved by the present invention as it relates to a drainage system for body fluids as defined in claim 1.

Another object of the present invention is to provide a fastening device that solves at least one of the above cited drawbacks in a simple and cost-effective manner.

The above said object is achieved by the present invention as it relates to a fastening device for a drainage system as defined in claim 10.

A preferred embodiment is hereinafter disclosed for a better understanding of the present invention by mere way of non-limitative example and with reference to the accompanying drawings, in which:
- figures 1 and 2 are perspective views with parts removed for clarity of a drainage system made according to the present invention and arranged in two different operative configurations;
- figure 3 is an axial sectional view of a fastening device made according to the present invention and used to lock the drainage system of figures 1 and 2 on the body of the patient; and
- figure 4 is an exploded perspective view of the fastening device of figure 3.

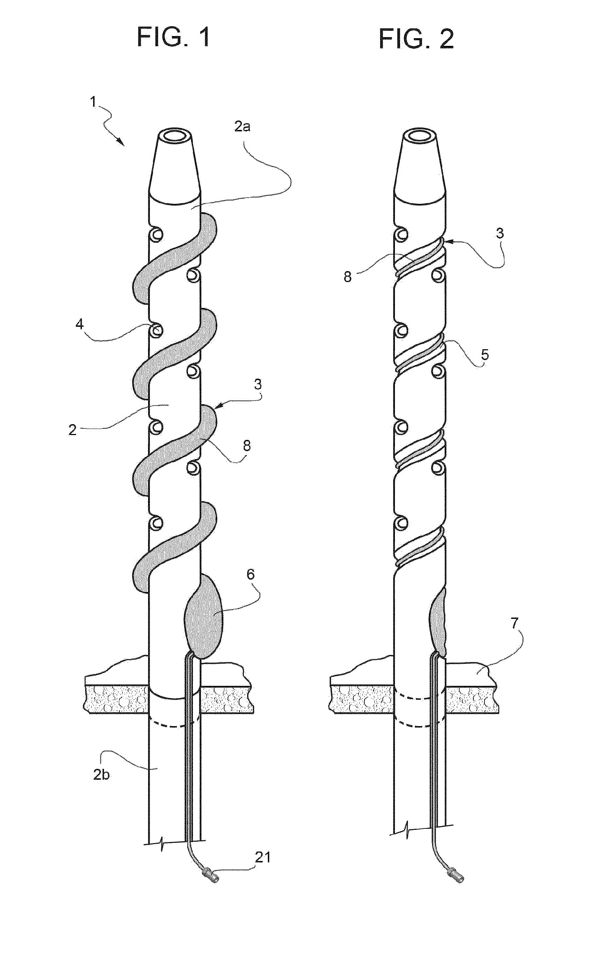

With reference to figures 1 and 2, numeral 1 indicates a drainage system for body fluids which substantially comprises:
- a catheter 2 having a distal segment 2a adapted to be inserted within the body of a patient and a proximal segment 2b arranged in use outside the body of the patient;
- a plurality of openings 4 for the passage of said fluids from the outside of catheter 2 to the inside of catheter 2; and
- a spacing device 3 of catheter 2 from the tissues of the patient.

Spacing device 3 comprises an expandable balloon 8 arranged helixwise about distal segment 2a laterally with respect to openings 4.

Catheter 2 can be made of silicon, polyvinyl chloride, polyurethane, polyethylene or any other suitable material.

Advantageously, catheter 2 defines, on the relative external surface, a groove 5 adapted to at least partially accommodate balloon 8. When balloon 8 is expanded, it will project radially with respect to the external surface of catheter 2, whereas when balloon 8 is collapsed, it will remain accommodated within groove 5 and the external surface of catheter 2 will result substantially smooth.

Balloon 8 also comprises a fixing portion 6 which, in a corresponding expanded condition, projects radially with respect to the external surface of catheter 2 so as to stably cooperate with the body of the patient. When drainage system 1 is inserted within the body of a patient and balloon 8 is expanded, fixing portion 6 is placed inside the body and prevents the extraction of catheter 2 from the body of the patient through the incision in skin 7 thereof.

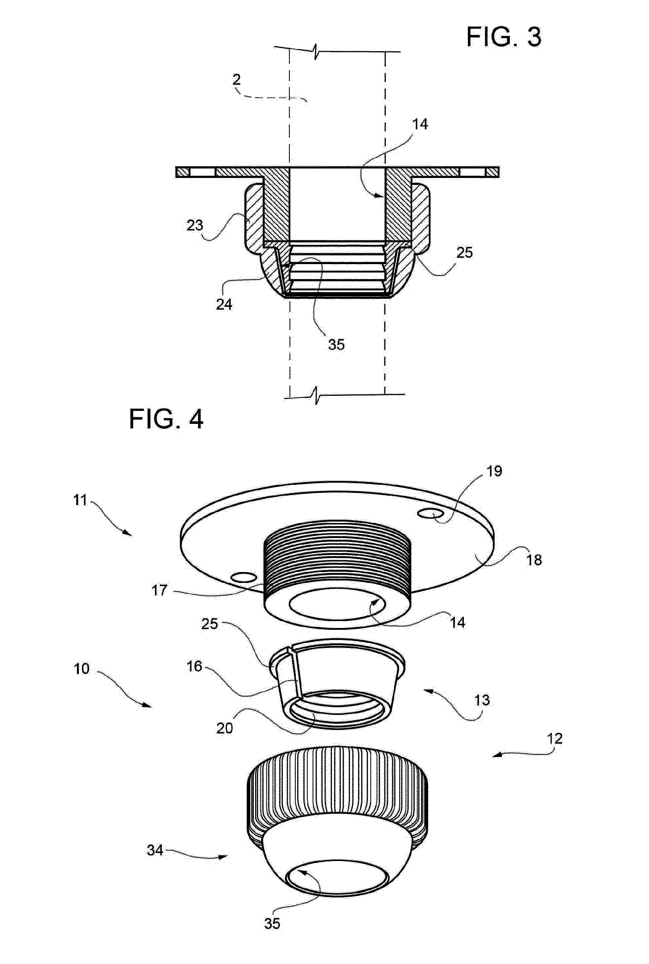

With reference to figures 3 and 4, drainage system 1 also comprises a fastening device 10 of catheter 2 to the body of the patient, which is extracorporeal and comprises in turn a fastening element 11 forming a through-seat 14 for inserting catheter 2 and connectable to skin 7 of the patient, and a retaining element 12 releasably coupleable to fastening element 11 to lock the position of catheter 2 on fastening element 11.

Fastening element 11 comprises an annular flange 18 provided with holes 19 for passing suture stitches for connecting to the body of the patient, and a bushing 17 axially protruding from a radially internal edge of flange 18 and defining an engagement portion coupleable with retaining element 12. Preferably, bushing 17 is externally threaded.

According to a preferred embodiment of the present invention, retaining element 12 comprises a cap 34 defining a through-seat 35 engageable in use by catheter 2 and an elastic ring 13 interposed between fastening element 11 and cap 34 and elastically loadable on the external surface of catheter 2 by effect of the coupling of cap 34 with fastening element 11.

Elastic ring 13 is preferably formed by a collar engageable by catheter 2 and provided with a transversal cut 16 which determines the elastic properties thereof, and by an annular flange 25 radially projecting from an end of the collar and axially available in use resting on the bushing 17 of fastening element 11.

Elastic ring 13 has an internal knurled cylindrical surface 20, and an external frustoconical surface tapered towards cap 34.

Elastic ring 13 is arranged in use with flange 25 resting on the axial end of bushing 17 opposite to the end from which flange 18 extends, and with the collar engaged within housing portion 24 of cap 34.

Internal surface 20 of elastic ring 13 is tapered towards retaining element 12.

Knurling 20 serves to lock catheter 2 and avoid the axial sliding through fastening device 10.

Cap 34 comprises an engagement portion 23 coupleable with bushing 17 of fastening element 11 and a housing portion 24 adapted to internally receive elastic ring 13, and having a smaller diameter than engagement portion 23. Engagement portion 23 is internally threaded so as to couple with bushing 17.

Cap 34 may have, at engagement portion 23, a corrugated outer portion so as to provide a better grip.

In use, the user places catheter 2 within the thorax of the patient by making an incision in skin 7 thereof and inserting catheter 2 by means of a pointed needle which passes through catheter 2. Catheter 2 is inserted in the thorax until distal segment 2a is completely within the thorax.

At this point, expandable balloon 8 may be expanded by the injection of a fluid, for example air, through a valve 21 placed at the end of balloon 8, which is outside the body of the patient. Thereby, the soft tissues surrounding distal segment 2a of catheter 2 are spaced from openings 4 and fluid to be drained is conveyed from the outside to the inside of catheter 2. The drainage can be enhanced by applying a negative pressure within catheter 2.

The expansion of balloon 8 also leads to fixing portion 6 projecting radially with respect to the external surface of catheter 2 and thus stably cooperating with the inner wall of the thorax of the patient. Thereby, drainage system 1 is stabilised and cannot come out from the thorax.

The fastening of catheter 2 to the body of the patient is achieved by expanding balloon 8, by applying fastening element 11 to the skin by suture stitches and by coupling retaining element 12 to fastening element 11.

Flange 25 of elastic element 13 is rested on the axial end of bushing 17. Then, cap 34 is fitted on bushing 17 and on elastic element 13. Engagement portion 23 is screwed on the threading of bushing 17 by determining the radial compression of elastic element 13 by housing portion 24. Such a radial compression brings internal knurled surface 20 in contact with the internal surface of catheter 2 thus locking catheter 2 within fastening device 10.

From an analysis of the features of drainage system 1 and of fastening device 10 according to the present invention, the advantages it allows to obtain are apparent.

In particular, in virtue of the fact that balloon 8 is arranged helixwise about distal segment 2a of catheter 2 laterally with respect to openings 4, openings 4 remain spaced from the soft tissues and are not obstructed. The helixwise arrangement also allows to arrange a greater number of openings 4 on catheter 2 and to better convey the fluids present in the thoracic cavity thus allowing a better drainage.

Further, the fact that balloon 8 is at least partially accommodated in a groove 5 obtained on the external surface of distal segment 2a of catheter 2 allows to obtain a drainage system which is smooth on the outside when balloon 8 is collapsed and therefore allows an easy insertion in the body of the patient.

The helixwise arrangement of balloon 8 about the catheter also allows to stably fix the catheter in a uniformly distributed manner within the body of the patient.

Moreover, in virtue of the fact that fastening element 11 of fastening device 10 is connectable directly to skin 7 of the patient, catheter 2 is prevented from having clearance. The incision in the skin allowing catheter 2 to be inserted in the thorax of the patient is therefore not torn apart. A possible clearance is also avoided by fixing portion 6 of balloon 8.

The presence of elastic ring 13 allows to tighten fastening device 10 without the risk of interfering with skin 7 of the patient and makes fastening device 10 adaptable to different diameters of catheter 2 by only modifying the size of elastic ring 13 and maintaining the size of fastening element 11 and cap 34 unchanged.

Finally, it is clear that modifications and variants to drainage system 1 and to fastening device 10 disclosed and shown herein can be made without departing from the scope of protection of the claims.

## Claims

1. A drainage system (1) for body fluids comprising:
- a catheter (2) having a distal segment (2a) adapted to be inserted within the body of a patient and a proximal segment (2b) arranged in use outside the body of the patient,
- a plurality of openings (4) for the passage of said fluids from the outside of the catheter (2) to the inside of the catheter (2),
- a spacing device (3) of the catheter (2) from the tissues of the patient, which comprises an expandable balloon (8) arranged helixwise about said distal segment (2a) laterally with respect to said openings (4), **characterised in that** said catheter (2) defines, on the relative external surface, a groove (5) adapted to at least partially accommodate said expandable balloon (8).

2. The drainage system according to claim 1, wherein said expandable balloon (8) comprises a fixing portion (6) which, in a relative expanded state, projects radially with respect to said external surface so as to stably cooperate with the body of the patient.

3. The drainage system according to claim 1 or 2, further comprising a fastening device (10) of the catheter (2) to the body of the patient, **characterised in that** the fastening device (10) is extracorporeal and comprises:
- a fastening element (11) forming a through-seat (14) for inserting the catheter (2) and connectable to the skin (7) of the patient,
- a retaining element (12) releasably coupleable to the fastening element (11) to lock the position of the catheter (2) on the fastening element (11).

4. The drainage system according to claim 3, **characterised in that** said fastening element (11) comprises an annular flange (18) provided with holes (19) for passing suture stitches for connecting to the body of the patient, and an annular engagement portion (17) axially protruding from said flange (18) and coupleable with the retaining element (12).

5. The drainage system according to claim 4, **characterised in that** the retaining element (12) comprises a cap (34) defining a through-seat (35) engageable in use by the catheter (2) and an elastic ring (13) interposed between the fastening element (11) and the cap (34) and elastically loadable on the external surface of the catheter (2) by effect of the coupling of the cap (34) with the fastening element (11).

6. The drainage system according to claim 5, **characterised in that** said elastic ring (13) comprises a collar engageable by said catheter (2), provided with a transversal cut (16) and axially available resting on the engagement portion (17) of the fastening element (11).

7. The drainage system according to claim 6, **characterised in that** said elastic ring (13) has an internal knurled cylindrical surface (20).

8. The drainage system according to claim 6 or 7, **characterised in that** said elastic ring (13) has an external frustoconical surface.

9. The drainage system according to any of claims 3 to 8, **characterised in that** said fastening element (11) and said retaining element (12) are coupleable by means of respective threadings.

10. A fastening device (10) for a drainage system (1) comprising:
- a fastening element (11) forming a through-seat (14) for inserting the catheter (2) and connectable to the skin (7) of the patient; and
- a retaining element (12) releasably coupleable to the fastening element (11) to lock the position of the catheter (2) on the fastening element (11).
